(19)

Europäisches Patentamt
European Patent Office
Office européen des brevets

(11) **EP 2 484 630 B1**

(12) **EUROPEAN PATENT SPECIFICATION**

(45) Date of publication and mention
of the grant of the patent:
**06.11.2019 Bulletin 2019/45**

(51) Int Cl.:
**B82Y 30/00** (2011.01)   **G01N 27/12** (2006.01)
**G01N 27/48** (2006.01)   **G01N 33/18** (2006.01)
**B01D 67/00** (2006.01)   **B01D 69/14** (2006.01)
**G01N 27/42** (2006.01)

(21) Application number: **11305112.2**

(22) Date of filing: **04.02.2011**

(54) **A Method for detecting and quantifying charged molecules by using in situ stripping voltammetry and use of a nanosensor**

Verfahren zum Einfangen geladener Moleküle mit in situ Strip-Voltamperemetrie und Verwendung eines Nanosensors

Procede pour capturer des molécules chargées via la voltamétrie de décapage et utilitsation d'un nanocapteur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**08.08.2012 Bulletin 2012/32**

(73) Proprietors:
• **École Polytechnique**
**91128 Palaiseau Cedex (FR)**
• **Centre National de la Recherche Scientifique**
**75016 Paris (FR)**
• **COMMISSARIAT À L'ÉNERGIE ATOMIQUE ET AUX ÉNERGIES ALTERNATIVES**
**75015 Paris (FR)**

(72) Inventors:
• **Wade, Travis Lee**
**75015 Paris (FR)**

• **Clochard, Marie-Claude Laurence**
**78500 Sartrouville (FR)**
• **Mezailles, Nicolas**
**91530 Saint Maurice Montcouronne (FR)**
• **Bessbousse, Haad**
**91120 Palaiseau (FR)**

(74) Representative: **Gevers & Orès**
**Immeuble le Palatin 2**
**3-5 Cours du Triangle**
**92036 Puteaux La Défense cedex (FR)**

(56) References cited:
EP-A1- 2 131 189    EP-A1- 2 230 511
WO-A1-2007/140252    WO-A2-2005/033685

• **CUSCITO ET AL: "Nanoporous beta-PVDF membranes with selectively functionalized pores", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, vol. 265, no. 1, 17 November 2007 (2007-11-17), pages 309-313, XP022349433, ISSN: 0168-583X**

EP 2 484 630 B1

**Description**

The invention relates to a method for capturing and quantifying charged molecules and favourably heavy metal ions in a fluid and to the use of a nanosensor for carrying out such a method. The present invention advantageously applies to sewage sludge or potable water, but it could apply to biological or medical fields. It could also apply to the detection of other ionic species in fluid, not only heavy metal ions.

The urban wastewater collection is one of the main facilities used for the disposal of commercial and domestic wastes, both of which may contain heavy metals. Wastewater treatment processes are generally effective in limiting the discharge of potentially toxic elements to the aquatic environment because a high proportion of the contaminant load is extracted and concentrated in the sewage sludge. Application of sewage sludge to agricultural land is the largest outlet for its beneficial use, and this is consistent with EC policy on waste recycling, recovery, and use. The Sewage Sludge Directive 86/278/EEC sets upper limits of trace metals in municipal sewage sludge for use on agricultural land, and revision of the directive is expected to lead to even more stringent limits. This is a critical issue due to the increasing amount of sludge produced (from 5.5M tonnes of dry matter in 1992 to 9M tonnes in 2005), and the fact that other alternatives (incineration and landfill) are not generally considered environmentally acceptable. Consequently, wastewater sludge quality must be protected and improved in order to secure the agricultural outlet as the most cost effective as sustainable solution.

The most widespread method for the determination of metal concentrations in wastewaters is via grab sampling and subsequent laboratory analysis. This method is both expensive, which limits its application, and time consuming, which means that pollution events can be missed, or detected too late. In the face of increasing levels of sludge production, the expected application of more stringent limits on heavy metal concentrations in sludge, and to identify, survey, and control the sources of input of toxic elements, there is a need for a low cost, time effective, easy to handle and very sensitive analysis system to determine the concentration of heavy metals in waste waters.

In addition, existing sensors to detect metal residues present following drawbacks:

- metal-based screen-print electrodes or modified glassy carbon are not accurate enough to detect residue (detection limits of 0.5 ($Pb^{2+}$), 2.0 ($Cu^{2+}$), 0.9($Hg^+$) and 1.4$\mu$g/L($Cd^{2+}$)),
- most of them suffer from analyte peak distortion, peak overlap, and loss of sensitivity due to interference of analyte ions with each other,
- Hg film coated electrodes are accurate enough at ppb levels ($\mu$g/L) by ion pre-concentration but not environmentally friendly.

Document WO 2009/147244 concerns a nanosensor for capturing heavy metal ions included in a fluid; said nanosensor comprising a functionalized radio grafted track-etched membrane (FRTEM) which contains nanopores; the membrane surfaces are metallised at both sides to serve as working electrode for an *ex situ* stripping voltammetry. The *ex situ* process comprises steps of immersing the nanosensor in the fluid containing heavy metal ions, removing, rising and immersing the nanosensor into a voltammetric cell to perform the stripping voltammetry, the voltammetric cell containing conductive fluid.

With the nanosensor as disclosed in WO 2009/147244 the nanosensor has to be displaced from the sample medium to a voltammetry cell.

The method of the present invention is based on the use of a nanosensor for capturing charged molecules, the nanosensor comprising:

- a functionalized radio grafted track-etched membrane (FRTEM) which contains polymer nanopores through at least two sides,
- a first electrode arranged on a first side of the functionalized radio grafted track-etched membrane (FRTEM), the first electrode is configured to serve as a working electrode for an *in situ* stripping voltammetry technique,
- a second electrode arranged on a second side of the functionalized radio grafted track-etched membrane (FRTEM), the second electrode is configured to serve as a counter electrode for an *in situ* stripping voltammetry technique. The nanosensor itself is not part of the present invention.

The nanosensor comprises electrodes designed on membrane surfaces for *in situ* analysis due to the ion exchange properties which make the membrane like a solid electrode with a finite resistance. Nanopores constitute low conductivity channels through which current can circulate between the working and counter electrodes.

The nanosensor allows for *in situ* analysis, which permits rapid monitoring of the efficiency of wastewater treatment and measure the charged molecules concentration in sewage sludge by functionalized radio grafted track-etched membranes (FRTEM), which are able to capture trace levels of for example heavy metal ions under wastewater flow by absorption.

The used nanosensor further comprises a third electrode arranged on a side of the functionalized radio grafted track-etched membrane (FRTEM), the third electrode being designed to serve as a reference electrode for a stripping voltammetry technique. All three electrodes are independent.

The membrane used in the method of the invention is based on a poly(vinylidenefluoride) PVDF film which can be radiografted with vinyl monomers. In other words, in the presence of vinyl monomer, such as acrylic acid (AA), a radical polymerization takes place by radiografting to specifically functionalize the nanopore wall with carboxylate hydrogel.

Advantageously, walls of nanopores are functionalized with one or a mixture of elements obtained from following groups:

- poly-acrylic acid,
- styrene,
- taurin,
- polyphosphine of $R_3P$ type,
- oxidized polyphosphine of $R_3P=0$ type.
- or other functional molecules known to specifically complex certain ions (for example carbamoyl-arms polyazamacrocyle which is known to specifically complex lead ions).

These groups may be used to functionalize the nanopores either by radiografting or coupling reactions.

More generally, depending on the specific functionalization of the nanopores, this nanosensor is useful for the analysis of biological or medical samples or any of the analysis that is performed by anodic stripping voltammetry (ASV) such as those of screen-printed electrodes and other stripping voltammetry techniques such as cathodic stripping voltammetry (CSV) or adsorptive stripping voltammetry (AdSV).

Preferably, the polymer nanopores consist in tubules through the entire thickness of the membrane. The membrane could be in the shape of a cylinder or parallelepiped in which tubules are all parallel and perpendicular to the two opposite sides of the membrane. The thickness of the membrane could substantially be of 9 $\mu$m and the nanopores could present a diameter between 20 and 200 nm. Moreover, each side of the membrane could present a diameter of substantially 4mm.

The membrane can be specifically grafted inside the nanopores. The synthesis of functionalized radio grafted membranes comprises notably steps of irradiation to activate radicals, chemical etching and radiografting. The remanence of the radicals within the nanopore walls after etching allows one to radiograft specifically inside the nanopores and not on the membrane surface. Under wastewater streams, for example, the functionalized radiografted, track-etched membrane (FRTEM) can thus trap cations and concentrate them.

The present invention explicitly refers to WO 2009/147244 which provides information relating to an ex situ analysis. The elements disclosed in WO 2009/147244 are incorporated in the present invention insofar as these elements are not incompatible with the teachings of the present invention.

Advantageously, walls of nanopores further contain bismuth ions, or other ions such as silver or selenium, which constitute an internal standard.

The absorbing ability of the functionalized track-etched nanoporous membranes is used to absorb a determined concentration of specific ions ($Bi^{3+}$, $Ag^+$, $Se^{4+}$ or $Co^{2+}$) to function as an internal standard for stripping analysis. The bismuth or silver or selenium or cobalt enhances the analysis of the other ions and is stripped last for quantitative comparison as an internal standard.

In order to improve adsorption and screening during stripping voltammetry, the nanosensor further comprises a first and a second piezoelectric contacts respectively on two opposite sides of the functionalized radio grafted track-etched membrane (FRTEM). These piezoelectric contacts can be driven by an alternating voltage.

In other words, voltages may be applied on piezoelectric contacts disposed on the functionalized radio grafted track-etched membrane (FRTEM) in order to deform said functionalized radio grafted track-etched membrane (FRTEM) during capture of charged molecules and/or stripping voltammetry analysis; the functionalized radio grafted track-etched membrane (FRTEM) being designed from a poly(vinylidenefluoride) PVDF film which is radio grafted with vinyl monomer and chelating molecule leading to a polymerization of nanopores walls. Piezoelectric properties of the membranes allow for ultrasonic agitation to enhance the filtering and analysis properties of the sensors.

In another aspect, the invention also enables the provision of a new method for detecting and quantifying charged molecules included in a fluid, said method comprising steps of:

- placing in the fluid a functionalized radio grafted track-etched membrane (FRTEM) which contains polymer nanopores, through two opposite sides; charged molecules being captured inside the polymer nanopores,
- applying an *in situ* stripping voltammetry analysis by using:
- a working electrode arranged on one side of the functionalized radio grafted track-etched membrane (FRTEM),
- a counter electrode arranged on a second side of the functionalized radio grafted track-etched membrane (FRTEM), and
- a reference electrode arranged in the fluid.

The present invention may advantageously be applied in detecting and quantifying charged molecules concentration of which is less than 5 ppb. Inside this range, the nanosensor presents an optimum sensitivity.

The invention also concerns a system for capturing charged molecules, the system comprising a processing unit equipped with several I/O plugs, each I/O plug comprising:

- a first connector to connect the working electrode,
- a second connector to connect the counter electrode, and
- a third connector to constitute a reference electrode in the fluid.

For the purpose of illustrating the invention, there is shown in the drawings a form that is presently preferred; it being understood, however, that this invention is not limited to the precise arrangements and instrumentalities.

Figure 1 is a schematic view illustrating Track-etch process: I) Swift Heavy Ions irradiation forms damaged zones in the polymer bulk along the ion pathway called latent tracks, II) the latent tracks are chemically etched forming nanopores of biconical shape at the early stage and III) if the track etch rate is sufficiently higher than the bulk etch rate, biconical nanopores become cylindrical;

Figure 2 is a graph illustrating the electron paramagnetic resonance (EPR) spectra per mass unit ($mg^{-1}$) for a heavy ion irradiated film;

Figure 3 is a graph illustrating the radical decay from EPR results versus time;

Figure 4 is a graph illustrating a FTIR spectra of swift heavy ion $_{58}Ni^{25+}$ irradiated PVDF-g-PAA for different etching times (AA concentration 100% v/v, fluence $10^9 cm^{-2}$, Mohr's salt 0.25% w/w, radiografting at 60°C for 1 hour); the maximum corresponds to O-C=O stretching (1701 $cm^{-1}$) and gives evidence of COOH groups radiografted PAA chains;

Figure 5 is a schematic view illustrating functionalization on nanosensor walls;

Figure 6 is a schematic view of a membrane cut in order to view nanopore walls;

Figure 7 shows a schematic cut-away side view of a membrane with gold electrodes on top and bottom sides;

Figure 8 shows a perspective view of the membrane of figure 6;

Figures 9-11 are schematic views illustrating the insertion of the membrane onto a membrane-holder constituting a nanosensor;

Figures 12a to 12d illustrate an online analysis in the detour of an effluent stream;

Figure 13 is a schematic view illustrating a handheld multi-way device comprising several input/outputs to connect several nanosensors according to the invention;

Figure 14 is a schematic view illustrating ions displacement through a nanopore wall during an *in situ* stripping voltammetry;

Figure 15 is a schematic view illustrating a nanosensor connected to a potentiostat according to the invention;

Figure 16 is a schematic view illustrating a nanosensor comprising a reference electrode and a working electrode disposed on one side, and a counter electrode disposed on another side;

Figures 17a to 17d illustrate curves concerning ASV calibration; and

Figure 18 is a schematic view illustrating a nanosensor comprising working electrode, counter electrode, and piezoelectric contacts for sonic agitation.

While the invention is susceptible to various modifications and alternative forms, specific embodiments thereof are shown by way of example in the drawings and will herein be described in detail. It should be understood, however, that the drawings and detailed description thereto are not intended to limit the invention to the particular form disclosed, but on the contrary, the intention is to cover all modifications, equivalents, and alternatives falling within the spirit and scope of the present invention as defined by the appended claims.

In accordance with the preferred embodiment, the method and device according to the invention relates to the following materials and processes:

**Materials:** Poly(vinylidenefluoride) (PVDF) films of 9 μm thickness provided by PiezoTech SA®, Saint Louis (France). Toluene, potassium hydroxide, potassium permanganate, potassium disulfite, acrylic acid (AA), Mohr's salt ($(NH_4)_2Fe(SO_4)_2.6H_2O$), sulphuric acid, EDC ($C_8H_{17}N_3.HCl$), phosphate buffer saline (PBS) and Alexa Fluor R 488 hydrazide (C21H15N4NaO10S2) purchased from Invitrogen®.

**Irradiation:** Prior to irradiation, PVDF films are toluene-extracted for 24 h. Swift heavy ion irradiation was performed at the GANIL, Caen (France). Films are irradiated with Kr ions (10.37 MeV/amu, fluence $10^7$ to $10^{10}$ $cm^{-2}$) under He atmosphere. In two cases, samples are stored at -20°C under $N_2$ atmosphere until chemical etching and radiografting.

**Chemical etching:** PVDF irradiated films are chemically etched using permanganate solution (0.25 M) in a highly alkaline medium (KOH 10 M) at 65°C with different etching times from 0.5 to 3 h. Membranes obtained are washed in potassium disulfide solution (15%) then dried at 50°C under vacuum.

**Radiografting:** PVDF films of initial size 20x20 $mm^2$, are weighed. The film was immersed at room temperature in a radiografting solution containing acrylic acid and Mohr's salt (0.25%w/w). After 15 minutes of bubbling nitrogen at room temperature, the sample is introduced into a thermostated water bath at 60°C for 1 hour. Membranes are washed with water and then Soxhlet-extracted in boiling water in order to extract free homopolymer. Functionalized membranes are dried at 50°C under vacuum.

**Infra-red spectroscopy:** FTIR spectra of PVDF films are obtained with a Nicolet Magna-IR 750 spectrometer equipped with a DGTS detector. Spectra are recorded in an attenuated total reflection mode (ATR) using a diamond-crystal with single reflection. Spectra are collected by accumulating 32 scans at a resolution of 2 $cm^{-2}$.

**Confocal Scanning Laser Microscopy (CSLM):** Measurements are performed at LLB (CEA-Saclay, France) with a Leica TCS-SP2 using an Ar laser (488 nm). Samples are observed in water with a 40x dry objective of numerical aperture 0.85.

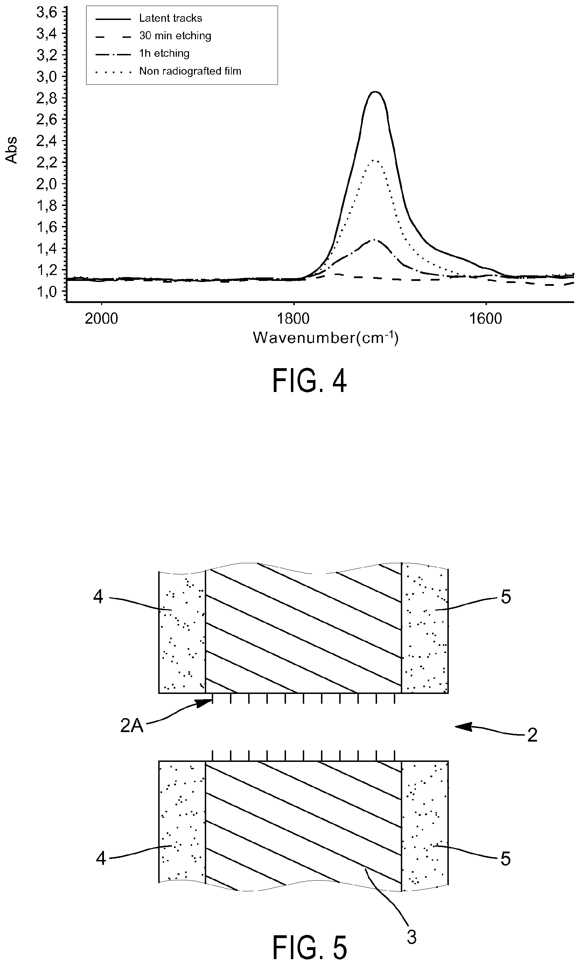

**Electrode fabrication,** as shown on figures 5-10: A first gold bottom layer, ~45nm is sputtered (EMITECH K550, UK) through a mask with 3 mm diameter holes. Next, a second gold layer, ~45 nm, is sputtered, through a mask, on the other side of the membrane over the same areas as the first gold area.

**Anodic stripping voltametry (ASV):** Electrochemical quantification of trace metal analytes is done by ASV. This involves the electrochemical reduction of the concentrated metal ions from the membrane on a metal coating on the surface of the membrane, which is the working electrode of an electrochemical cell. The electrode is held at a very negative potential, which reduces all of the trapped metal ions to their metallic, zero valence, state. The electrode is then scanned slowly toward positive potentials where the ions are oxidized, anodically stripped, and the resulting current is proportional to the concentrations of the ions trapped in the membrane. Different metals oxidize at different potentials so the kind of metal can be identified. The analysis is performed with a potentiostat (HEKA PG310, Germany) with a Ag/AgCI (Metrohm, Switzerland) reference electrode.

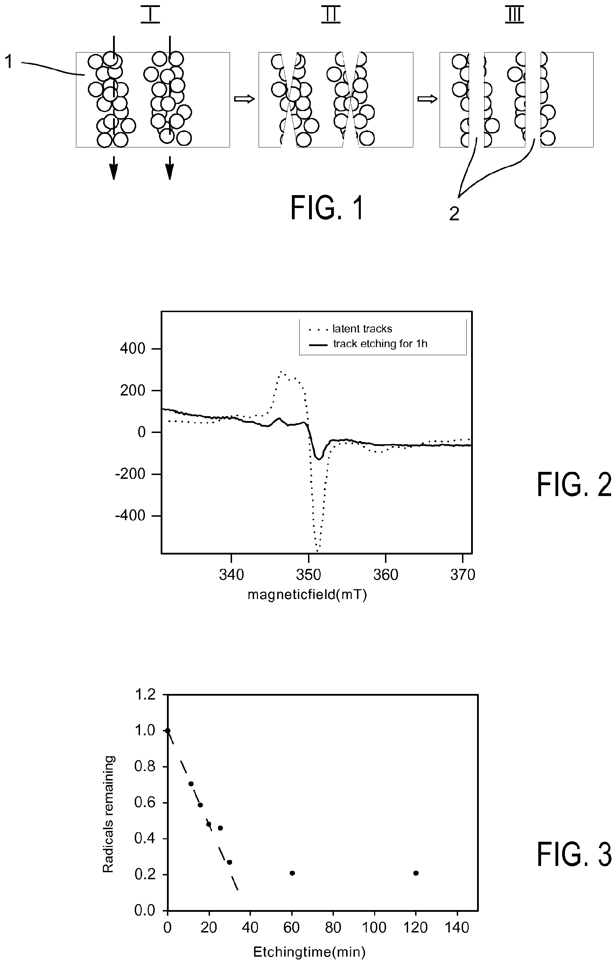

The synthesis of functionalized radio grafted membranes is described according to figures 1-4. With respect to figure 1, a track-etch process is described. This process comprises three steps:

I) latent track formation along the ion pathway through a polymer film 1,
II) symmetrical attack of latent tracks by hydrolysis at early stage of the process, and
III) formation of cylindrical pores 2.

In other words, for the preparation of a functionalized radio grafted track-etched membrane 3 (FRTEM), polymer film 1 is first bombarded by swift heavy ions and the formed tracks along the ion passage are revealed under alkaline chemical treatment.

The obtained nanoporous polymer membrane 3 does not need to undergo a subsequent e-beam irradiation to increase radical proportion in polymer bulk submicronic pore diameter. Indeed, after etching times inferior to one hour, the radical residues within nanopore walls were found sufficiently numerous to persue a radiografting from the pore walls.

Figure 2 shows an electron paramagnetic resonance (EPR) spectra per mass unit ($mg^{-1}$) for a heavy ion irradiated film before etching (latent tracks) and after 1 hour of track etching. In the latter case, the non-horizontal baseline is due to paramagnetic impurities ($KMnO_4$ from the etching bath). Figure 3 shows the radical decay from EPR results versus time. Radicals are always presents even after 1 hour of track etching.

Concerning the functionalization, in the presence of vinyl monomer, such as acrylic acid (AA), a radical polymerization takes place by radiografting process to specifically functionalize the nanopore walls with carboxylate hydrogel as shown from FTIR spectra in figure 4. This polymerization is specific to the nanopore walls and does not occur on the membrane surface. The selectivity of the grafting is checked by labelling of the amine pre-functionalized surface of the PVDF nanoporous membrane by a fluorescent probe specific to the amines and the poly(acrylic acid) inside the pores by a second fluorescent probe specific to the acid groups.

Functionalization is usually accomplished by absorbing functional molecules onto the electrode surface, applying a carbon paste, dipping the electrodes in a solution of the functionalizing agent, or spin coating of a functional polymer such as nafion. The nanoporous PVDF membranes can be functionalized to serve as modified electrodes for stripping voltammerty.

The carboxylate hydrogel located inside the nanopores chelates weakly with transition metal ions. In order to improve the membrane ion affinity, it is possible to further functionalize it by other radical or common coupling reactions with other chelatant molecules.

Chelation ("chelation" in French) is the binding or complexation of a mono or bi- or multidentate ligand. These ligands, which are often organic compounds, are called chelants, chelators, chelating agents, or sequestering agent. These molecules should have a very important chelating power to trap even more effectively the heavy metal ions.

These molecules should have different chelating powers or specificities in order to trap even more effectively or selectively the metal ions or other ions ( Mono- and polyacides, mono and polyamines, Ethylene Diamine Tetra Acetate and derivatives, cyclodextrines, crownethers or any clathrates complexes).

In the schematic example of figure 5 a nanosensor 9 is represented. Element 2A represent radicals of chelatant molecules used to functionalize nanopore walls. Figure 5 illustrates metallised (gold, carbon or other metal) surfaces 4, 5 and PVDF nanoporous membrane 3. The functionalization, however, takes place before the metallization.

As an example, in order to bring sulfonic acid ion exchange functionalization to the pores the radiografting can for example be performed with 100% styrene (instead of acrylic acid) at 60°C for 30 minutes in a nitrogen atmosphere. The $-C_6H_4SO_3^-$ functionalized pores are strong cation exchange with high capacity that might be more effective for weakly adsorbed ions such as Co, Cr, and Fe. The membranes are then put in a 0.5 M chlorosulfonic acid ($SO_2Cl$) in dichloromethane solution at room temperature under a nitrogen atmosphere for 30 minutes. After, it is then possible to hydrolize the membrane in two stages: NaOH 1M during 2 hours then $H_2SO_4$ 1M during 1 hour.

As another example to impart sulfonate ($-SO_3^-$

) functionalization to the poly (acrylic acid) functionalized PVDF membranes, a coupling reaction can be performed with Taurin (poly(PPy-co-PPYCONHtaurin)). Taurin coupled to acrylic acid functionalized pores (poly(PPy-co-PPYCONH taurin) due to the -NH groups might have anion exchange effects which could help for metal oxyanions such as $AsO_4^{3-}$, $HAsO_4^{2-}$ or $AsO_3^{3-}$. The membranes may react with $10^{-2}$ molar ethyl-3-(3-dimethylamino-propyl)carbodiimide, $C_8H_{17}N_3 \cdot HCL$ (EDC) and 3.2 x $10^{-2}$ molar Taurin $C_2H_7O_3NS$ for 24 h in aqueous medium.

The chemical nature of the polymer that is radiografted in the nanopores will have a direct impact on the metal(s), and the amount of metal, to be trapped and analyzed. It is interesting to have access to a variety of polymers with tunable coordinative abilities to metal centers. The polyphosphine (of $R_3P$ type) polymers are for example well tailored for electron rich metal centres such as Au, Cu, Pt and Zn, whereas the oxidized version (of $R_3P=O$ type) are more efficient for electron poor metal centres such as Co, Cr, Fe, and V. Polymers containing at the same time the two moieties can also be synthesized.

The functionalized nanoporous PVDF membranes may be pre-dosed with $Bi^{3+}$ (or other ions such as $Ag^+$ or $Se^{4+}$ or $Co^{2+}$) for stripping analysis. Bismuth has a large window for voltammetric analysis and is insensitive to dissolved oxygen in sample mediums.

Bismuth is nontoxic and may be integrated inside nanopores as follows: a functionalized, non metallized, nanoporous PVDF membrane is submerged in a specific concentration of bismuth ion solution, $Bi^{3+}$, for a set time, for example 1 ppb Bi solution for 15 mn. The membrane is then removed from the $Bi^{3+}$ solution, rinsed, dried, and metallized. The $Bi^{3+}$ doped membrane is then configured as a stripping voltammety sensor and placed in a sample medium to absorb metal ions, $M^{n+}$.(ex. $Cd^{2+}$, $Pb^{2+}$). The stripping voltammetry is normally performed to yield a current/potential plot. The currents peaks can be integrated and their concentration can be determined by comparison with the area of the bismuth peak. This internal standard scheme could be used as a means for controlled dosing of ions into systems. Also, $Ag^+$ or $Se^{4+}$ or $Co^{2+}$ could be used in the same manner as an internal standard.

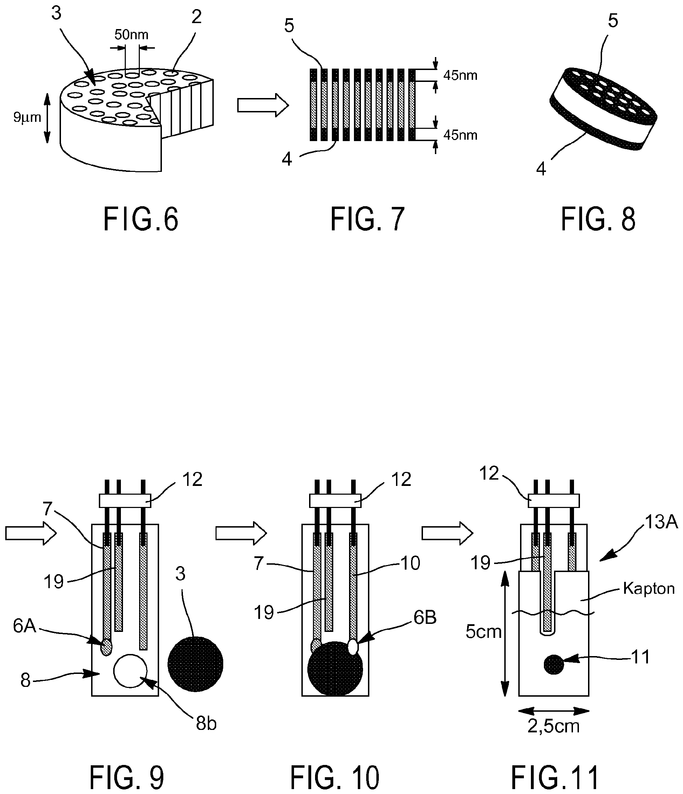

Turning now to figures 6-11, a nanosensor 13 is shown. The nanosensor is based on the membrane 3, the shape of which is a cylinder of 9 $\mu$m thick and of 4.0 mm diameter, with $10^6$ to $10^{10}$ nanopores per $cm^2$ of 50 nm diameter. Once the membrane has been functionalized, gold is sputtered on each side of the membrane 3 in figure 7 : a bottom electrode 4 of 45nm thick, preferably a value from 30 to 50nm, and a top electrode 5 of 45nm thick, preferably a value from 30 to 50nm,. The sputtering is made in such a way that the nanopores remain open. The gold layers can cover or not the entire side surface. On figure 8 the perspective view of the membrane shows the top electrode 5 and the bottom electrode 4. On figures 9-11, the bottom layer 4 is contacted with silver paste 6A to copper contacts 7 lithographically patterned on 1.5 $cm^2$ Plexiglas placket 8 with a 4mm diameter hole, 8b. The top layer 5 is contacted with silver paste 6B to other copper contacts 10 on the same placket 8. The membrane 3 is then covered with waterproof tape, Kapton, 3M, except for a 2 mm diameter circular area 11 in the centre of the membrane, which will serve as a window for exposure of the membrane to the wastewater, see figure 11. The diameter of area 11 is less or equal to the diameter of the hole 8b.

Preferably, also electrode 19 is not covered by the waterproof tape so that said electrode 19 is in contact with wastewater.

The copper leads are not completely covered by the tape and remain outside of the solution so they can be connected to a potentiostat via a bridge 12.

For the stripping voltrammetry, electrode 4 is the working electrode; electrode 5 is the counter electrode. A reference electrode 19 can be realised, but not necessary, on the membrane 3. On figures 9-11, reference electrode 19 is not sputtered on the membrane 3 but is on the Plexiglas packet 8 so that it will be inside the fluid during a stripping voltrammetry.

The nanosensor 13, 13A is very compact and easy to handle.

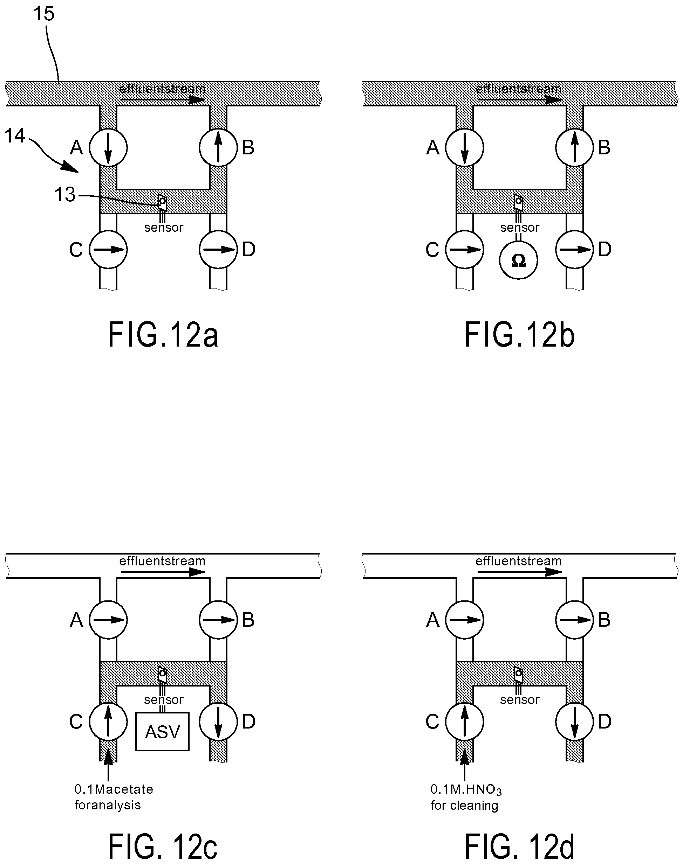

The nanosensor 13, 13A can then be immersed into wastewater for a preset time to absorb ions based on calibration. According to an embodiment of the invention, the nanosensor could be used by placing it in a bypass circuit of the fluid or for example in a small detour 14 of a waste-water effluent pipe 15 as shown on figure 12a. The detour 14 comprises an inlet pipe equipped with an inlet valve A, and an outlet pipe equipped with an outlet valve B. Between said valves, is disposed the nanosensor 13, 13A. The detour 14 also comprises two evacuation pipes equipped respectively with valves C and D.

Figures 12 a-d illustrate an on-line analysis. In figure 12a), valves A and B open and valves C and D closed, the nanosensor is in the detour 14 of the effluent stream. In this mode the sensor can be absorbing ions for a calibrated time. In figure 12b), valves A and B open and valves C and D closed, the sensor could be in a conductivity mode for constantly measuring the resistance of the effluent. The conductivity measurements are discussed hereafter. The conductivity mode could serve as an alarm for pollution events such as a dramatic increase in effluent conductivity. In figure 12c, valves A and B closed and valves C and D open, a supporting electrolyte could be injected into the sensor chamber and the sensor could be connected to a potentiostat for ASV analysis. In figure 12d), valves A and B closed and valves C and D open, a cleaning solution, 0.1 M $HNO_3$ or EDTA, could be injected into the sensor chamber after analysis in-order to clean the sensor for reuse.

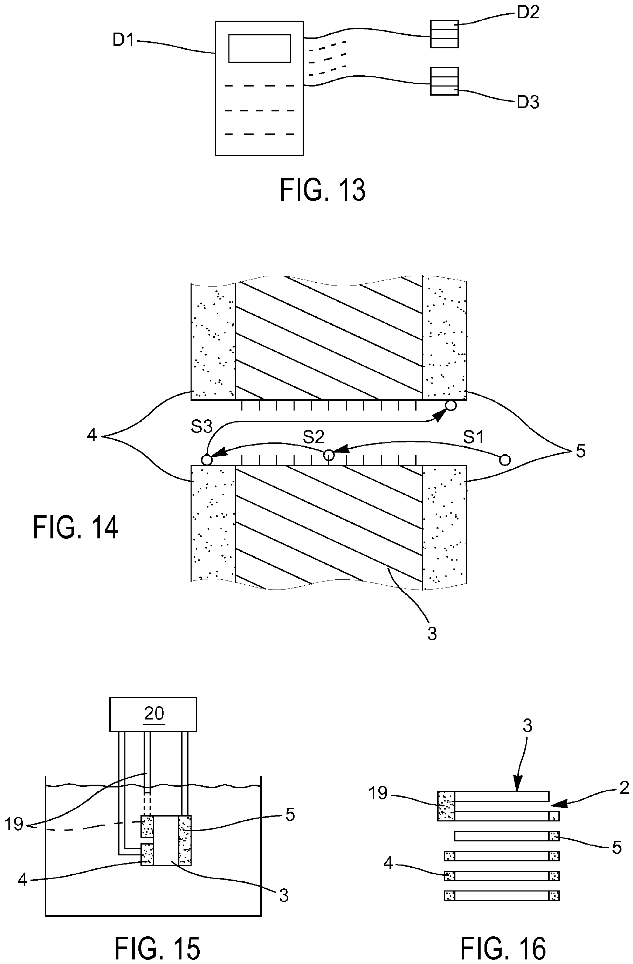

Figure 13 illustrates a mobile device D1 for capturing charged molecules. This device D1 comprises several input/outputs to connect several nanosensors D2, D3. One input/output can receive a nanosensor functionalized with any suitable molecule. The device may be connected to different nanosensors functionalized with different molecules respectively. Thus, each nanosensor may be adapted to detect a specific ion. Device D1 contains a processing unit controlling one or a plurality of potentiostats which feed nanosensors D2, D3.

For stripping voltammetry it is necessary to use three electrodes: a working electrode, a reference electrode, and a counter electrode. The potential is controlled between the working electrode and the reference electrode and the current is measured between the working electrode and the counter electrode.

Since the current is measured between the working and counter electrodes the ionic transport is between the working and counter electrodes. The problem with *in situ* measurements is that the ionic concentration of the sample medium is uncontrolled and often very low. This hampers current response because of the high resistance. The functionalized nanoporous PVDF membrane is designed as a conducting or ion exchange medium between the working and counter electrodes in order to allow for *in situ* voltammetry. More precisely, when the membrane is placed into a sample medium, the ions of interest are absorbed from the sample medium to nanopore walls as stated by S1 figure 14. To perform an in situ stripping voltammetry, a potentiostat is used to control the working, counter and reference electrodes while the membrane stays in the sample medium. At step S2 on figure 14, ions migrate to the working electrode 4 by a negative potential -1200 mV applied between the reference electrode 19 and the working electrode 5. Ions are then reduced onto the electrode 4 at the potential of -1200 mV. At step S3, the potential is then scanned in a positive sense from -1200 mV to +400 mV, which oxidizes (dissolves) the ions into the sample medium and creates a current which is carried through the membrane between the working electrode 4 and the counter electrode 5. Advantageously the current passes through the nanopores. The resulting current intensity and position, measured between the working and the counter electrodes 4, 5 of the membrane 3 indicate the type and concentration of the heavy metal ions. The resulting currents are proportional to the contaminate ions concentration based on previous calibration.

Figure 15 shows the membrane attached by wires to pin connectors for connection to a potentiostat. Since the counter and working electrodes are both gold they can be interchanged to suit different situations. The reference electrode 19 is connected to the potentiostat 20 and its free end is either freely disposed in the sample medium (figure 15) or metallised on a part of a side of the membrane as shown on figure 16 (figure 15 dashed line). With the membrane configuration shown in figure 15, the functionalized membrane functions to absorb ions from a sample medium and the current for analysis is carried by the membrane functionalization groups between the working and counter electrodes and the potential is controlled between the working and reference electrodes. In this way, even high resistance mediums can be measured, even non liquid mediums could be analysed such as air or exhaust.

A use of the nanosensor according to the present invention concerns *in situ* conductivity measurements. Preferably, two sensors may be used, the first sensor including a functionalized radio grafted track-etched membrane (FRTEM) which is positively functionalized, preferably with a poly(PPy-co-PPYCONHtaurin), the second sensor including a functionalized radio grafted track-etched membrane (FRTEM) which is negatively functionalized, preferably with a poly(acrylic acid).

In general, a resistance, $R(\Omega)$ of a medium depends on the size and shape of the measuring cell and the resistivity of the medium. For a uniform cell with an area, $A$ ($m^2$), and length, $L(m)$ the resistivity, $\rho(\Omega m)$, is:

$$\rho = RA/L$$

The conductivity of a cell, $\kappa$ ($Sm^{-1}$; where $S = 1/\Omega$), is the reciprocal of the resistivity:

$$\kappa = L/RA$$

To have fast and stable measurements of mediums with low conductivity, i.e. high resistance, it is convenient to have a cell with a small separation between electrodes, $L$, and a large area of overlap, $A$. This then can be used to define a cell constant ($\gamma$):

$$\gamma = L/A$$

The nanosensor is used for *in situ* conductivity measurements of low conductivity mediums such as pure water and air because the electrodes are very close together providing a very low cell constant, y. The nanosensor increases the concentration of ions near the electrodes because of the functionalization of the nanopores and the improvement of the hydrophilicity of the nanopores due to the functionalization and thus increases the sensitivity of the measurements. This is very important for conductivity analysis since it is usually done *in situ* in very low conductivity media such as pure water. As it is possible to functionalize the membrane nanopore walls with different chemicals in order to make the adsorption more specific, it would be useful to have two sensors: one with positively charged functions more sensitivity to anions (such as poly(PPy-co-PPYCONH taurin) and another one with negatively charged functions more specific to cations (such as polyacrylic acid).

As a matter of fact, conductivity measurements may be made continuously in real time or after a delay of immersion, depending for example on the resistivity of the solution to measure. If the solution is not conductive enough to perform an efficient analysis, a conductive electrolyte may be added into the solution.

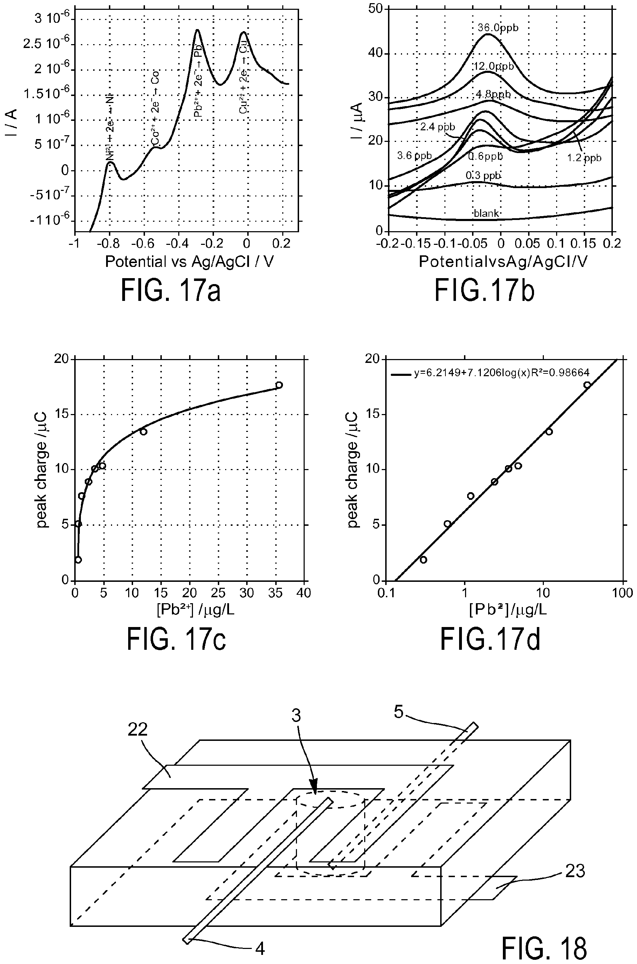

Figure 17a) illustrates an ASV analysis according to the invention after immersion in 10 ml of a stirred 8.4 ppb ($\mu$g/L) Ni, 24.8 ppb Co, 23.8 ppb Pb, and 9.9 ppb Cu multi-ion ion solution for 1 hour. Figure 17b) illustrates Square-wave ASV curves according to the invention after immersion in 50 ml of a stirred $Pb^{2+}$ ion solution for 30 minutes in different concentrations. The curve from a blank analysis is also plotted. Figure 17c) illustrates a Square wave ASV calibration curve for $Pb^{2+}$ ions. Concentrations as low as 0.1 ppb (0.1 $\mu$g/L) have been repeatedly measured. Figure 17d) shows a log - charge plot of the calibration curve demonstrating Temkin isotherm adsorption behaviour of the PAA functionalised $\beta$-PVDF nanopores.

According to another embodiment the nanosensor used in the method of the present invention comprises piezoelectric contacts. Piezoelectric effect arises from the application of an electric field to a poled (stretched) materials such as the PVDF polymer family: polyvinylidene fluoride PVDF, P(VDF-TrFE) or terpolymers of PVDF such as P(VDF-TrFE-CFE). The electric field causes length, tensile, thickness, and shear deformation. Ultrasonic transducers have been made with PVDF, designed according to the present invention, that operate at kHz frequency.

Ultrasonic agitation enhances the diffusion of solutions through membranes and enhances solution transport. This is used to improve the efficiency of filtration if the membranes are made of piezoelectric materials such as the PVDF family of polymers.

Sonic agitation can break up sediments and micelles and disassociate complexes and precipitates to release contaminants for stripping voltammetry. With piezoelectric effects incorporated into the nanosensor, its sensitivity is improved and it is more useful for *in situ* applications such as rivers and lakes where the pollutant ions are tied up in sediments, micelles, complexes and precipitates and in general improve solubility.

Sonic agitation cycle may be added to the stripping voltammetry protocol during the extraction of adsorbed ions from the membrane to the cathode surface and stopped for the anodic stripping cycle. This could shorten the extraction time and improving its efficiency. This could aid the cleaning of the membrane for reuse. Another sonic agitation cycle could be added to the beginning of the adsorption cycle in order to release trapped ions and then stopped in order to allow the ions to be adsorbed by the membrane.

The nanosensor as described on figure 18 contains nanopores area 3 localized to the centre by the use of masks during the irradiation of the piezoelectric polymers. The piezoelectric electrodes 22, 23 may be by sputtering or evaporation through a mask but are not required to be very thin as the electrodes 4 and 5.

The method and the use of the present invention are useful for the analysis of biological or medical samples or any of the analysis that is performed by stripping voltammetry such as those of screen-printed electrodes. The ability to configure the functionalized nanoporous PVDF membrane for in situ analysis makes it potentially useful in non liquid mediums such as air or exhaust. The internal standard scheme could be used as a means for controlled dosing of ions.

Numerous variations and modifications will become apparent to those skilled in the art once the above disclosure is fully appreciated. It is intended that the following claims be interpreted to embrace all such variations and modifications.

**Claims**

1. Method for detecting and quantifying charged molecules included in a fluid, said method comprising steps of:

   - placing in the fluid a nanosensor comprising a functionalized radio grafted track-etched membrane (FRTEM) which contains polymer nanopores;
   a first electrode arranged on a first side of the functionalized radio grafted track-etched membrane (FRTEM), and
   a second electrode arranged on a second side of the functionalized radio grafted track-etched membrane (FRTEM),
   - capturing charged molecules of the fluid inside the polymer nanopores,
   and
   - applying an *in situ* stripping voltammetry analysis by using:

      - said first electrode as working electrode,
      - said second electrode as counter electrode, and
      - a reference electrode arranged in the fluid.

2. Method according to claim 1, **characterized in that** the stripping voltammetry analysis is an anodic stripping voltammetry (ASV), a cathodic stripping voltammetry (CSV), or an Adsorptive Stripping Voltammetry (AdSV).

3. Method according to claim 1 or 2, **characterized in that** walls of nanopores further contain a predetermined concentration of bismuth or silver ions or selenium ions or cobalt ions which constitute an internal standard.

4. Method according to any of preceding claims 1-3, **characterized in that** voltages are applied on piezoelectric contacts disposed on the functionalized radio grafted track-etched membrane (FRTEM) in order to deform said functionalized radio grafted track-etched membrane (FRTEM) during capture of charged molecules and/or stripping voltammetry analysis; the functionalized radio grafted track-etched membrane (FRTEM) being designed from a poly(vinylidenefluoride) PVDF film which is radio grafted with vinyl monomer and chelating molecule leading to a polymerization of nanopores walls.

5. Method according to any of preceding claims 1-4, **characterized in that** the functionalized radio grafted track-etched membrane (FRTEM) is placed in a fluid in a bypass circuit.

6. Use of a nanosensor for *in situ* conductivity and in situ stripping voltammetry measurements, the nanosensor comprising :

   - a functionalized radio grafted track etched membrane (FRTEM) which contains polymer nanopores,
   - a first electrode arranged on a first side of the functionalized radio grafted track etched membrane (FRTEM),
   - a second electrode arranged on a second side of the functionalized radio grafted track etched membrane (FRTEM), wherein

   in one mode
   the first electrode and the second electrode are configured for conductivity measurements,
   and in another mode
   the first electrode is configured as a working electrode and the second electrode is configured as a counter electrode for an *in situ* stripping voltammetry technique.

7. Use according to claim 6 **characterized in that**, the nanosensor further comprises a third electrode arranged on a side of the functinalized radio grafted track-etched membrane (FRTEM), the third electrode being designed to serve as a reference electrode for a stripping voltammetry technique.

8. Use according to claim 6 or 7 **characterized in that** walls of nanopores are functionalized with one or a mixture of elements obtained from following groups :

   - poly-acrylic acid,
   - styrene,
   - taurin,
   - polyphosphine of $R_3P$ type,
   - oxidized polyphosphine of $R_3P=0$ type,
   - other functional molecules known to specifically complex certain ions.

9. Use according to claims 6 to 8 **characterized in that** walls of nanopores further contain bismuth ions or silver ions or selenium ions or cobalt ions which constitute an internal standard.

10. Use according to claim 6 to 9 **characterized in that** the nanosensor further comprises a first and a second piezoelectric contacts respectively on two opposite sides of the functionalized radio grafted track etched membrane (FRTEM).

11. Use according to claim 10, characterize in that two sensors are used, the first sensor including a functionalized radio grafted track-etched membrane (FRTEM) which is positively functionalized, preferably with a poly(PPy-co-PPYCONHtaurin), the second sensor including a functionalized radio grafted track-etched membrane (FRTEM) which is negatively functionalized, preferably with a poly(acrylic acid).

12. Use of the method according to claims 1-5, in detecting and quantifying charged molecules concentration of which is less than 5 ppb.

**Patentansprüche**

1. Verfahren zum Nachweis und zur Quantifizierung von geladenen Molekülen, die in einem Fluid enthalten sind, wobei das Verfahren die folgenden Schritte umfasst:

   - Platzieren, in dem Fluid, eines Nanosensors, der eine funktionalisierte strahlengepfropfte Kernspur-Membran (FRTEM) umfasst, die polymere Nanoporen enthält;
   eine erste Elektrode, die auf einer ersten Seite der funktionalisierten strahlengepropften Kernspur-Membran (FRTEM) angeordnet ist, und eine zweite Elektrode, die auf einer zweiten Seite der funktionalisierten strahlengepropften Kernspur-Membran (FRTEM) angeordnet ist,
   - Auffangen von geladenen Molekülen des Fluids innerhalb der polymeren Nanoporen und
   - Anwenden einer *in situ*-Analyse mittels Stripping-Voltammetrie unter Verwendung:

      - der ersten Elektrode als Arbeitselektrode,
      - der zweiten Elektrode als Gegenelektrode,
      - einer im Fluid angeordneten Referenzelektrode.

2. Verfahren nach Anspruch 1, **dadurch gekennzeichnet, dass** die Analyse mittels Stripping-Voltammetrie eine anodische Stripping-Voltammetrie (ASV), eine kathodische Stripping-Voltammetrie (CSV) oder eine adsorptive Stripping-Voltammetrie (AdSV) ist.

3. Verfahren nach Anspruch 1 oder 2, **dadurch gekennzeichnet, dass** Wände von Nanoporen weiter eine vorbestimmte Konzentration von Wismut- oder Silberionen oder Selenionen oder Kobaltionen enthalten, die einen internen Standard darstellen.

4. Verfahren nach einem der vorstehenden Ansprüche 1-3, **dadurch gekennzeichnet, dass** Spannungen an piezoelektrischen Kontakten angelegt werden, die auf der funktionalisierten strahlengepfropften Kernspur-Membran (FRTEM) angeordnet sind, um die funktionalisierte strahlengepfropfte Kernspur-Membran (FRTEM) während des Auffangens von geladenen Molekülen und/oder der Analyse mittels Stripping-Voltammetrie zu verformen; wobei die funktionalisierte strahlengepfropfte Kernspur-Membran (FRTEM) aus einer Poly(vinylidenfluorid)-PVDF-Folie ausgebildet ist, die mit Vinylmonomer und chelatbildendem Molekül strahlengepfropft ist, was zu einer Polymerisation von Nanoporenwänden führt.

5. Verfahren nach einem der vorstehenden Ansprüche 1-4, **dadurch gekennzeichnet, dass** die funktionalisierte strahlengepfropfte Kernspur-Membran (FRTEM) in ein Fluid in einem Bypass-Kreislauf platziert wird.

6. Verwendung eines Nanosensors für *in situ*-Leitfähigkeits- und in situ-Stripping-Voltammetriemessungen, wobei der Nanosensor umfasst:

   - eine funktionalisierte strahlengepfropfte Kernspur-Membran (FRTEM), die Polymernanoporen enthält,
   - eine erste Elektrode, die auf einer ersten Seite der funktionalisierten strahlengepropften Kernspur-Membran (FRTEM) angeordnet ist,
   - eine zweite Elektrode, die auf einer zweiten Seite der funktionalisierten strahlengepropften Kernspur-Membran (FRTEM) angeordnet ist, wobei in einem Modus die erste Elektrode und die zweite Elektrode für Leitfähigkeitsmessungen konfiguriert sind,

   und in einem anderen Modus
   die erste Elektrode als Arbeitselektrode und die zweite Elektrode als Gegenelektrode für eine in situ-Stripping-Voltammetrietechnik konfiguriert ist.

7. Verwendung nach Anspruch 6, **dadurch gekennzeichnet, dass** der Nanosensor weiter eine dritte Elektrode umfasst, die auf einer Seite der funktionalisierten strahlengepfropften Kernspur-Membran (FRTEM) angeordnet ist, wobei die dritte Elektrode ausgebildet ist, um als Referenzelektrode für eine Stripping-Voltammetrietechnik zu dienen.

8. Verwendung nach Anspruch 6 oder 7, **dadurch gekennzeichnet, dass** Wände von Nanoporen mit einem oder einer Mischung von Elementen funktionalisiert sind, die aus folgenden Gruppen erhalten werden:

   - Polyacrylsäure,
   - Styrol,
   - Taurin,
   - Polyphosphin vom Typ $R_3P$,
   - oxidiertes Polyphosphin vom Typ $R_3P=0$,
   - sonstige funktionelle Moleküle, die dafür bekannt sind, bestimmte Ionen spezifisch zu komplexieren.

9. Verwendung nach Ansprüchen 6 bis 8, **dadurch gekennzeichnet, dass** Wände von Nanoporen weiter Wismutionen oder Silberionen oder Selenionen oder Kobaltionen enthalten, die einen internen Standard darstellen.

10. Verwendung nach Anspruch 6 bis 9, **dadurch gekennzeichnet, dass** der Nanosensor weiter einen ersten und einen zweiten piezoelektrischen Kontakt jeweils auf zwei gegenüberliegenden Seiten der funktionalisierten strahlengepfropften Kernspur-Membran (FRTEM) umfasst.

11. Verwendung nach Anspruch 10, **dadurch gekennzeichnet, dass** zwei Sensoren verwendet werden, wobei der erste Sensor eine funktionalisierte strahlengepfropfte Kernspur-Membran (FRTEM) beinhaltet, die positiv funktionalisiert ist, vorzugsweise mit einem Poly(PPy-co-PPYCONHtaurin), wobei der zweite Sensor eine funktionalisierte strahlengepfropfte Kernspur-Membran (FRTEM) beinhaltet, die negativ funktionalisiert ist, vorzugsweise mit einer Poly(acrylsäure).

12. Verwendung des Verfahrens nach Ansprüchen 1-5 zum Nachweis und zur Quantifizierung einer Konzentration von geladenen Molekülen, deren Konzentration weniger als 5 ppb beträgt.

**Revendications**

1. Procédé de détection et de quantification de molécules chargées incluses dans un fluide, ledit procédé comprenant les étapes consistant à :

   - placer dans le fluide un nanocapteur comprenant une membrane gravée sur piste fonctionnalisée par radiogreffage (FRTEM) qui contient des nanopores polymères ;
   une première électrode agencée sur un premier côté de la membrane gravée sur piste fonctionnalisée par radiogreffage (FRTEM) et

une deuxième électrode agencée sur un second côté de la membrane gravée sur piste fonctionnalisée par radiogreffage (FRTEM),
- capturer des molécules chargées du fluide à l'intérieur des nanopores polymères, et
- appliquer une analyse de voltammétrie par redissolution in situ en utilisant :

    - ladite première électrode comme électrode de travail,
    - ladite deuxième électrode comme contre-électrode, et
    - une électrode de référence agencée dans le fluide.

**2.** Procédé selon la revendication 1, **caractérisé en ce que** l'analyse de voltammétrie par redissolution est une voltammétrie par redissolution anodique (ASV), une voltammétrie par redissolution cathodique (CSV) ou une voltammétrie par redissolution après absorption (AdSV).

**3.** Procédé selon la revendication 1 ou 2, **caractérisé en ce que** les parois des nanopores contiennent en outre une concentration prédéterminée d'ions de bismuth ou d'argent ou d'ions de sélénium ou d'ions de cobalt qui constituent un standard interne.

**4.** Procédé selon l'une quelconque des revendications précédentes 1 à 3, **caractérisé en ce que** des tensions sont appliquées sur des contacts piézoélectriques disposés sur la membrane gravée sur piste fonctionnalisée par radiogreffage (FRTEM) afin de déformer ladite membrane gravée sur piste fonctionnalisée par radiogreffage (FRTEM) au cours de la capture de molécules chargées et/ou de l'analyse de voltammétrie par dissolution ; la membrane gravée sur piste fonctionnalisée par radiogreffage (FRTEM) étant conçue à partir d'un film de poly(fluorure de vinylidène) PVDF qui est radiogreffage avec un monomère de vinyle et une molécule chélatante menant à une polymérisation des parois de nanopores.

**5.** Procédé selon l'une quelconque des revendications précédentes 1 à 4, **caractérisé en ce que** la membrane gravée sur piste fonctionnalisée par radiogreffage (FRTEM) est placée dans un fluide d'un circuit de dérivation.

**6.** Utilisation d'un nanocapteur pour des mesures de conductivité in situ et de voltammétrie par redissolution in situ, le nanocapteur comprenant :

    - une membrane gravée sur piste fonctionnalisée par radiogreffage (FRTEM) qui contient des nanopores polymères,
    - une première électrode agencée sur un premier côté de la membrane gravée sur piste fonctionnalisée par radiogreffage (FRTEM),
- une deuxième électrode agencée sur un second côté de la membrane gravée sur piste fonctionnalisée par radiogreffage (FRTEM), dans lequel :

    dans un mode
    la première électrode et la deuxième électrode sont configurées pour des mesures de conductivité
    et dans un autre mode
    la première électrode est configurée comme électrode de travail et la deuxième électrode est configurée comme contre-électrode pour une technique de voltammétrie par redissolution in situ.

**7.** Utilisation selon la revendication 6, **caractérisée en ce que** le nanocapteur comprend en outre une troisième électrode agencée sur un côté de la membrane gravée sur piste fonctionnalisée par radiogreffage (FRTEM), la troisième électrode étant conçue pour servir d'électrode de référence pour une technique de voltammétrie par redissolution.

**8.** Utilisation selon la revendication 6 ou 7, **caractérisée en ce que** les parois des nanopores sont fonctionnalisées avec un élément ou un mélange d'éléments obtenus à partir des groupes suivants :

    - acide polyacrylique,
    - styrène,
    - taurine,
    - polyphosphine de type $R_3P$,
    - polyphosphine oxydée de type $R_3P = 0$,
    - d'autres molécules fonctionnelles connues pour complexer spécifiquement certains ions.

**9.** Utilisation selon les revendications 6 à 8, **caractérisée en ce que** les parois de nanopores contiennent en outre des ions de bismuth ou des ions d'argent ou des ions de sélénium ou des ions de cobalt qui constituent un standard interne.

**10.** Utilisation selon les revendications 6 à 9, **caractérisée en ce que** le nanocapteur comprend en outre un premier et un second contact piézoélectrique, respectivement, sur les deux côtés opposés de la membrane gravée sur piste fonctionnalisée par radiogreffage (FRTEM).

**11.** Utilisation selon la revendication 10, **caractérisée en ce que** deux capteurs sont utilisés, le premier capteur incluant une membrane gravée sur piste fonctionnalisée par radiogreffage (FRTEM) qui est fonctionnalisée de manière positive, de préférence avec une poly(PPy-co-PPYCONHtaurine), le second capteur incluant une membrane gravée sur piste fonctionnalisée par radiogreffage (FRTEM) qui est fonctionnalisée de manière négative de préférence avec un acide polyacrylique.

12. Utilisation du procédé selon les revendications 1 à 5 dans la détection et la quantification de molécules chargées dont la concentration est inférieure à 5 ppb.

FIG. 1

FIG. 2

FIG. 3

FIG. 4

FIG. 5

FIG.6

FIG. 7

FIG. 8

FIG. 9

FIG. 10

FIG.11

FIG.12a

FIG.12b

FIG. 12c

FIG. 12d

FIG. 13

FIG. 14

FIG. 15

FIG. 16

FIG. 17a

FIG.17b

FIG. 17c

FIG.17d

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009147244 A